# EUROPÄISCHE PATENTSCHRIFT

(11) **EP 0 849 595 B1**
(45) Veröffentlichungstag und Bekanntmachung des Hinweises auf die Patenterteilung: **09.05.2001**
(21) Anmeldenummer: 96120421.1
(22) Anmeldetag: 18.12.1996
(51) Int. Cl.: G01N 33/543

(54) **Synthetische Partikel als Agglutinationsreagenzien**
Use of synthetic particles as reagents in agglutionation reactions
Utilisations des particules synthétiques comme réactif d'agglutination

(43) Veröffentlichungstag der Anmeldung: 24.06.1998
(73) Patentinhaber: STIFTUNG FÜR DIAGNOSTISCHE FORSCHUNG, CH-1785 Cressier sur Morat (CH)
(72) Erfinder: Schwind, Peter, 1700 Fribourg (CH); Bashforth, David, 2003 Neuchâtel (CH); Hobbs, Roderick N., Chester, Cheshire CH2 2AX (GB); Margetts, Graham, Shrewsbury, Shropshire SY1 4JH (GB); Marshall, Michael J., Oswestry, Shropshire SY11 4AR (GB); Roberts, Mark J.J., Shrewsbury, Shropshire SY3 8AD (GB)
(74) Vertreter: Weiss, Wolfgang, Dipl.-Chem. Dr.

(56) Entgegenhaltungen:
- EP-A- 0 158 443
- EP-A- 0 279 525
- EP-A- 0 305 337
- EP-A- 0 348 174
- US-A- 4 021 534
- US-A- 5 552 064

## Beschreibung

Die vorliegende Erfindung betrifft ein Verfahren zum Nachweis eines Analyten in einer Probeflüssigkeit durch Agglutination, wobei man die Probeflüssigkeit mit einem Agglutinationsreagenz und einer inerten Matrix in Kontakt bringt, die Reaktionsmischung dem Einwirken von Gravitation aussetzt und die Reaktion zwischen dem Analyten und dem Agglutinationsreagenz bestimmt. Weiterhin werden neue Reagenzien zur Durchführung des erfindungsgemäßen Verfahrens offenbart.

Methoden zum Nachweis von Analyten durch Hämagglutinationssowie Partikelagglutinationstests sind bekannt. Diese Tests kommen vor allem in der Diagnostik infektiöser Krankheiten zur Antigen- bzw. Antikörperdetektion zum Einsatz. Alle diese Methoden haben jedoch den gemeinsamen Nachteil, daß sie zeitund personalaufwendig sind und daß die Ergebnisse oft nur sehr schwer interpretiert werden können.

Für Hämagglutinationstests, bei denen unfixierte Erythrozyten als Agglutinationsreagenzien verwendet werden, sind Gel-Immunoassayverfahren bekannt, bei denen Hämagglutinate durch einen Zentrifugationsschritt durch eine inerte Matrix von einzelnen nicht-agglutinierten Erythrozyten getrennt werden (vgl. z.B. EP-A-0 194 212, EP-A-0 305 337, EP-A-0 557 546, EP-A-0 634 216, EP-A-0 485 228 und WO95/31731). Gemäß diesen Verfahren werden agglutinierte Erythrozyten auf oder in der inerten Matrix festgehalten und können damit deutlich von nicht-reagierenden einzelnen Erythrozyten getrennt werden, welche die Matrix durchdringen können und am Boden des Reaktionsgefäßes sedimentieren.

Einzig in EP-A-0 305 337, die auf eine Prioritätsanmeldung aus dem Jahr 1987 zurückgeht, findet sich die Feststellung, daß als Agglutinationsreagenzien auch synthetische Partikel, wie Latex oder polymerisierte Agarose verwendet werden können. Angaben über die Eigenschaften derartiger synthetischer Partikel finden sich jedoch nicht. Außerdem wurde dieser Vorschlag in der folgenden Zeit weder in Patenten noch in anderen Publikationen aufgegriffen. Im Gegenteil, wird in neueren Veröffentlichungen, wie EP-A-0 557 546, auf unfixierte Erythrozyten als Agglutinationsreagenzien abgestellt.

Ein Nachteil von unfixierten Erythrozyten besteht jedoch in ihrer Instabilität, die unter bestimmten Reaktionsbedingungen zu einer Hämolyse führt. Diese Instabilität hat oft unerwünscht kurze Verfallsdaten von Produkten auf Basis unfixierter Erythrozyten zur Folge. Weiterhin ist eine standardisierte und reproduzierbare Herstellung von Erythrozytenpräparationen, insbesondere von Antikörper- oder Antigen-gekoppelten Erythrozytenpräparationen, nur mit großem Aufwand möglich. Somit ist eine ausreichende Uniformität der physikalischen Eigenschaften bei Erythrozytenpräparationen praktisch ausgeschlossen.

Eine der vorliegenden Erfindung zugrundeliegende Aufgabe bestand somit darin, die obengenannten Nachteile, die sich durch die Verwendung von Erythrozyten als Agglutinationsreagenzien ergeben, mindestens teilweise zu beseitigen. Insbesondere sollen durch die vorliegende Erfindung synthetische Partikel bereitgestellt werden, die als Agglutinationsreagenzien eingesetzt werden können und in bekannten Gel-Immunoassays das Verhalten von Erythrozyten zu simulieren vermögen. Weiterhin sollen die synthetischen Partikel eine einfache Kopplung biologischer Substanzen erlauben und so beschaffen sein, daß sie als Agglutinationsreagenzien den bisher verwendeten Erythrozyten hinsichtlich der Sensitivität und Spezifität mindestens gleichwertig sind. Diese synthetischen Partikel sollen in Agglutinationsverfahren eingesetzt werden, die einfacher durchzuführen und auszuwerten sind als bisher bekannte Verfahren.

Diese Aufgabe wird gelöst durch ein Verfahren zum Nachweis eines Analyten in einer Probeflüssigkeit durch Agglutination, wobei man die Probeflüssigkeit mit einem Agglutinationsreagenz und einer inerten Matrix in Kontakt bringt, die Reaktionsmischung dem Einwirken von Gravitation aussetzt und die Reaktion zwischen dem Analyten und dem Agglutinationsreagenz bestimmt, dadurch gekennzeichnet, daß man als Agglutinationsreagenz synthetische Partikel verwendet, deren Durchmesser und Dichte so gewählt werden, daß sie sich gegenüber der Matrix im wesentlichen wie Erythrozyten verhalten.

Überraschenderweise wurde festgestellt, daß sich, wenn gleichzeitig die Dichte und der Durchmesser optimal eingestellt werden, tatsächlich synthetische Partikel herstellen lassen, die in einem Gel-Immunoassay problemlos eine inerte Matrix passieren können und damit frische Erythrozyten perfekt simulieren.

Im Rahmen der Unternehmungen, die zur vorliegenden Erfindung führten, zeigte sich, daß die Passage rigider Polymerpartikel im Vergleich zu Erythrozyten (Durchmesser 6 - 8 *µ*m) stärker verzögert wird. Unter gegebenen Standardbedingungen bedeutete das konkret, dass kommerziell erhältliche Standardpartikel mit einem Durchmesser von 3 - 7.5 *µ*m nur unvollständig in die Gelmatrix sedimentierten, während grössere (11.9 *µ*m) bzw. kleinere synthetische Partikel (< 1 *µ*m) nur schwach in das Gel eindringen konnten. Eine Erhöhung der Parameter Zentrifugationsdauer (von 10 min bis auf 50 min) bzw. Zentrifugationsgeschwindigkeit (von 1030 bis 1300 rpm) brachte zwar eine bessere, aber immer noch keine vollständige Sedimentation. Selbst wenn durch diese beiden Massnahmen eine optimale Sedimentation hätte erreicht werden können, so ist doch eine Veränderung/Erhöhung der Zentrifugationsdauer und -geschwindigkeit aus folgenden Gründen sehr nachteilig:
1. Das erfindungsgemässe System soll ein im Vergleich zu vergleichbaren Methoden besonders zeitunaufwendiges Verfahren erlauben. Bei einer möglichen Zentrifugationsdauer von 50 Minuten würde sich die angestrebte Testdauer von 20 Minuten inklusive Inkubation damit verdreifachen.
2. Wie dem Fachmann bekannt ist, verringert sich die Sensitivität in Gel-Zentrifugationsverfahren mit steigender Zentrifugationsgeschwindigkeit.
3. Bei Einhaltung der vorgegebenen Parameter steht für das erfindungsgemässe System eine kommerziell erhältliche Zentrifuge zur Verfügung.

Überraschenderweise wurde festgestellt, daß mit synthetischen Partikeln von geringerem Durchmesser als dem von Erythrozyten und höherer Dichte als üblich, ein den Erythrozyten vergleichbares Verhalten bei der Passage der inerten Matrix erzielt werden kann. Dabei ist sowohl der Gebrauch von sphärischen als auch von asymmetrischen synthetischen Partikeln möglich.

Als wesentlich für das erfindungsgemäße Verfahren haben sich synthetische Partikel mit einem mittleren Durchmesser ≤ 5 *µ*m und insbesondere von 1 bis 5 *µ*m erwiesen. Besonders bevorzugt liegt der mittlere Durchmesser zwischen 2 und 4 *µ*m. Die spezifische Dichte der Partikel ist grösser als oder gleich 1,1 g/cm³ und liegt günstigerweise im Bereich von 1,1 bis 1,8 g/cm³. Bevorzugt liegt die spezifische Dichte im Bereich von 1,1 bis 1,6 g/cm³ und am meisten bevorzugt im Bereich von 1,15 bis 1,4 g/cm³, insbesondere wenn das Nachweisverfahren bei Standardbedingungen, wie sie für bekannte kommerzielle Erythrozyten-Gel-Immunoassays (z. B. DiaMed) vorgegeben sind, durchgeführt wird.

Vorzugsweise sind die im erfindungsgemäßen Verfahren als Agglutinationsreagenz verwendeten synthetischen Partikel organische Polymer- oder Copolymerpartikel. Besonders bevorzugte Materialien sind Styrol und Styrolderivate wie etwa Bromstyrole und insbesondere Copolymere davon. Die Herstellung uniformer Polymerpartikel im Größenbereich, der für die erfindungsgemäßen Agglutinationsreagenzien in Frage kommt, sind dem Fachmann hinlänglich bekannt (Arshady (1992): Suspension, emulsion and dispersion polymerization: A methodological Survey. Colloid & Polymer Science 270, 717-732; Okubo und Shiozaki (1992): Production of micron-size monodispense polymer particles by seeded polymerization utilizing dynamic swelling method with cooling process. Polymer International 30, 469-474). Derartige Methoden bedienen sich üblicherweise einer Kombination aus Emulsions- und Dispersionspolymerisierung, um Partikel mit exakt definierten physikalischen Eigenschaften herzustellen.

Für die visuelle Detektion der Agglutinationsreaktion im erfindungsgemäßen Verfahren können Farbstoffe, insbesondere wasserunlösliche Farbstoffe, in die Partikel inkorporiert werden. So erweisen sich blau-, gelb-, grün-, schwarz- oder rotgefärbte synthetische Partikel als sehr gut geeignet zur visuellen Auswertung. In einer besonders bevorzugten Ausführungsform der vorliegenden Erfindung wird wegen ihrer besonders guten Detektierbarkeit mit roten Partikeln gearbeitet.

Die Robustheit der synthetischen Partikel ermöglicht die Immobilisierung einer großen Anzahl unterschiedlicher Liganden nach bekannten industriellen Standardmethoden auf ihrer Oberfläche. Zweckmäßigerweise werden dabei Ligandenmoleküle immobilisiert, die mit dem zu bestimmenden Analyten bindefähig sind. Die Ligandenmoleküle können über adsorptive, kovalente oder hochaffine Wechselwirkungen immobilisiert werden. Die kovalente Kopplung kann beispielsweise über chemisch reaktive Gruppen, die auf die Oberfläche exponiert sind, erfolgen. Beispiele für solche Gruppen sind Carboxyl- Amino-, Aldehydund Expoxygruppen. Die Immobilisierung über hochaffine Wechselwirkungen wird durch zwei Partner eines hochaffinen Bindepaares vermittelt, wie etwa Streptavidin bzw. Avidin/Biotin, Hapten/Antikörper, Saccharid/Lectin etc. Mittels dieser kovalenten und hochaffinen Wechselwirkungen können Ligandenmoleküle, wie etwa Peptide, z.B. synthetisch hergestellte Peptide, Proteine, z.B. Glycoproteine, Lipoproteine, rekombinante Polypeptide, Immunglobuline, Nucleinsäuren, z.B. DNA oder RNA, Nucleinsäureanaloga, Saccharide, z.B. Mono-, Di-, Oligo- und Polysaccharide, Lipide, Hormone, Metaboliten oder andere biologische Substanzen immobilisiert werden. Die Immobilisierung kann dabei direkt oder durch Verwendung von Linkern erfolgen, um auf kontrollierte Weise eine bevorzugte optimale Orientierung der gebundenen Ligandenmoleküle zu erzielen.

Ebenso ist aber auch eine Immobilisierung von Ligandenmolekülen über rein adsorptive Prozesse möglich. Dies ist beispielsweise für Zellmembranen, wie etwa Membranen von Erythrozyten, Thrombozyten oder Leukozyten, Fragmente von Zellmembranen oder Lysate von Zellen oder Pathogenen, wie etwa Viren, Bakterien oder Parasiten, möglich. Bei Verwendung gefärbter synthetischer Partikel ist eine zusätzliche Einfärbung von Membranen überflüssig.

Das erfindungsgemäße Verfahren betrifft den Nachweis eines Analyten in einer Probeflüssigkeit. Als Probeflüssigkeit dienen vorzugsweise Körperflüssigkeiten, die gegebenenfalls verdünnt sein können, z.B. Blut, Serum, Plasma, Saliva oder Urin. Das Volumen der Probeflüssigkeit für das erfindungsgemäße Verfahren kann in weiten Bereichen variieren, vorzugsweise werden für Mikrotests Volumina von 1 bis 200 *µ*l verwendet.

Als Agglutinationsreagenz werden im erfindungsgemäßen Verfahren die beschriebenen synthetischen Partikel verwendet. Diese Partikel enthalten auf ihrer Oberfläche vorzugsweise ein spezifisch und mit hoher Affinität mit dem Analyten bindefähiges Ligandenmolekül. Das Agglutinationsreagenz enthält üblicherweise mehrere Bindungsstellen für den Analyten, wodurch das Entstehen von vernetzten Agglutinationskomplexen aus Analyt und Agglutinationsreagenz ermöglicht wird.

Die durch das erfindungsgemäße Verfahren nachweisbaren Analyten sind Substanzen, die eine spezifische und hochaffine Wechselwirkung mit dem Agglutinationsreagenz eingehen können, z.B. Antigene bzw. Antikörper, die durch eine Immunreaktion bestimmt werden können, oder auch Nucleinsäuren, die durch eine Hybridisierungsreaktion bestimmt werden können. Eine erste bevorzugte Ausführungsform der vorliegenden Erfindung betrifft den Nachweis von Antikörpern als Analyten in der Probeflüssigkeit, z.B. Antikörper gegen Pathogene, wie etwa Viren (HIV, Hepatitis-Viren), Bakterien oder Protozoen, Antikörper gegen Autoantigene, Antikörper gegen Tumoren oder Antikörper gegen Allergene.

Weiterhin gelingt durch das erfindungsgemäße Verfahren auch ein klassenspezifischer Nachweis von Antikörpern, z.B. eine Unterscheidung zwischen IgG- und IgM-Antikörpern, was bei der Diagnostik z. B. infektiöser Erkrankungen und Autoimmun-Er-krankungen oftmals von erheblicher Bedeutung ist.

Andererseits können durch das erfindungsgemäße Verfahren auch Antigene bestimmt werden, z.B. freie Antigene, wie etwa Serumproteine, Metaboliten, Hormone, Mediatoren etc. oder trägergebundene Antigene, wie etwa zelluläre Blutgruppenantigene etc.

Beim erfindungsgemäßen Verfahren wird ein Reaktionsgefäß mit einer Matrix verwendet, so daß nach Einwirkung von Gravitationskräften eine qualitative oder semiquantitative Bestimmung der Agglutinationsreaktion zwischen dem zu bestimmenden Analyten und dem Agglutinationsreagenz ermöglicht wird. Obwohl die Einwirkung von Gravitationskräften auch durch längere Sedimentation verursacht werden kann, verwendet man vorteilhafterweise eine Zentrifugation, da schon nach kurzer Zeit die gewünschte Sedimentation bewirkt werden kann. Die optimalen Bedingungen hinsichtlich der Zentrifugationsdauer und g-Zahl kann der Fachmann ohne Schwierigkeiten für jedes Analysesystem ermitteln. Diese Bedingungen werden insbesondere durch die Beschaffenheit des Agglutinationskomplexes zwischen Agglutinationsreagenz und Analyt, der Komponenten des Reaktionsgemisches in ungebundenem Zustand sowie der jeweils verwendeten Matrix bestimmt.

Vorzugsweise wird beim erfindungsgemäßen Verfahren eine inerte partikelförmige Matrix verwendet. Andererseits kann auch beispielsweise die in EP 96 10 428.3 beschriebene kompakte Matrix eingesetzt werden.

Vorzugsweise ist die Matrix eine inerte partikelförmige Matrix. Mit dem Merkmal "inert" kommt zur Geltung, daß die Matrix keine unspezifische Reaktion mit dem Analyten bzw. dem Agglutinationsreagenz eingehen darf. Vorzugsweise werden als Matrix Partikel verwendet, wie sie im Handel für die Flüssigkeitschromatographie (z.B. von Merck, Pharmacia, Bio-Rad, Tosohaas) erhältlich sind. Spezifische Beispiele sind Sephadex® , Sepharose® , Sephacryl® , Bio-Gel® oder Toyopearl® . Es handelt sich dabei um Produkte auf der Basis von vernetzten Polymeren oder Copolymeren, wie Agarose, Polyacrylamid, Polydextran, Styrol/Divinylbenzol oder Polymethacrylat. Ebenfalls in Frage kommen Glaskügelchen. Die Korngrößen der Partikel der Matrix betragen vorzugsweise 10 *µ*m bis 200 *µ*m. Ebenfalls in Frage kommen Matrizes, an die bereits Nachweisreagenzien, wie etwa Antikörper, gekoppelt sind, wie sie etwa von den Firmen Pierce oder Pharmacia erhältlich sind. Der Fachmann kann durch einfache Vorversuche bestimmen, ob die Partikel für eine bestimmte Nachweismethode brauchbar sind.

Die Interpretation der Ergebnisse des erfindungsgemäßen Verfahrens ist derart, daß nach Einwirkung von Gravitationskräften auf das Reaktionsgemisch
a) bei einer starken Agglutination das Reaktionsprodukt aus dem zu bestimmenden Analyten und dem Agglutinationsreagenz nicht oder nur sehr gering in die Matrix eindringen kann,
b) bei einer schwachen Agglutination das Reaktionsprodukt in die Matrix eindringt, sie aber nicht vollständig durchdringen kann und
c) beim Fehlen einer Agglutination die im Reaktionsgefäß enthaltenen Komponenten die Matrix im wesentlichen vollständig durchdringen können.

In einer bevorzugten Ausführungsform des erfindungsgemäßen Verfahrens wird mit zwei oder mehreren unterschiedlichen Arten von synthetischen Partikeln als Agglutinationsreagenzien gearbeitet. Es gibt nämlich verschiedene, miteinander assoziierte Krankheiten, bei denen mit herkömmlichen Testverfahren zunächst ein sogenanntes Screening durchgeführt werden muß, bei dem etabliert wird, ob die untersuchte Person Antikörper gegen eine zu dieser Gruppe gehörende Krankheit trägt. Ist die getestete Körperflüssigkeit der untersuchten Person im Screening-Test positiv, so muß sie nach herkömmlichen Methoden in einem zweiten Schritt einem sogenannten Differenzierungstest unterzogen werden, bei dem dann festgestellt wird, gegen welche der Krankheiten der Gruppe die Antikörper gerichtet sind. Ein typisches Beispiel für diese Art des Vorgehens stellt die HIV-Diagnostik dar. Im Screening wird festgestellt, ob HIV-spezifische Antikörper vorliegen. In der Differenzierung wird festgestellt, ob diese gegen HIV-I, gegen HIV-II oder HIV-I und HIV-II gerichtet sind.

Beim erfindungsgemäßen Verfahren kann nunmehr mit zwei verschiedenen Arten von synthetischen Partikeln gearbeitet werden, die sich vorzugsweise nicht in Größe, Form, Dichte und Flexibilität unterscheiden, aber eine unterschiedliche Färbung, z.B. Rot und Blau, und eine unterschiedliche Ausstattung mit Oberflächenliganden aufweisen. Da aus diesen unterschiedlichen Spezies von synthetischen Partikeln eine homogene Suspension mit gleichen Anteilen jeder Spezies gebildet wird, kann diese Suspension wie für einen Einzeltest benutzt werden. Die resultierenden Ergebnisse können dann wie folgt interpretiert werden: Liegen beide zu bestimmenden Analyten in der Probeflüssigkeit vor, so ergibt sich nach Zentrifugation eine rote und eine blaue Bande auf der Matrix oder in deren oberem Bereich. Wenn nur ein Analyt enthalten ist, kann im oberen Bereich der Matrix eine Bande mit der entsprechenden Farbe detektiert werden, während die synthetischen Partikel mit der anderen Farbe die Matrix durchdringen und unterhalb davon angeordnet sind. Sind weder der erste noch der zweite Analyt in der Probeflüssigkeit enthalten, bildet sich unter der Matrix eine Schicht aus beiden synthetischen Partikeln aus. Entsprechend können natürlich auch drei oder mehrere verschiedene synthetische Partikel mit unterschiedlichen Farben eingesetzt werden, um die entsprechende Anzahl von Parametern gleichzeitig zu differenzieren.

Zur Durchführung des Verfahrens können an sich beliebige Reaktionsgefäße verwendet werden, die zum Einsatz in einem Gel-Agglutinationstest geeignet sind. Das Volumen der Reaktionsgefäße ist vorzugsweise von 50 *µ*l bis 2 ml. Die Reaktionsgefäße sind vorzugsweise mit einem Trichter zur Aufnahme der Reagenzien ausgestattet. In einer bevorzugten Ausführungsform wird eine Anordnung von mehreren Reaktionsgefäßen verwendet, die zusammen auf einer Karte oder Scheibe angeordnet sind. Diese auf einer Karte oder Scheibe befindlichen Reaktionsgefäße können zum Nachweis derselben Analyten oder zum Nachweis unterschiedlicher Analyten vorgesehen sein.

Beispielsweise können zur Durchführung des erfindungsgemäßen Verfahrens die von der Fa. DiaMed für die Blutgruppenanalyse vertriebenen Karten verwendet werden, auf denen 6 Mikroreaktionsgefäße angeordnet sind. Ebenso können auch Produkte der Firmen Ortho und Diagast verwendet werden.

Die Einwirkung der Gravitation auf das Reaktionsgemisch erfolgt vorzugsweise durch Zentrifugation der Reaktionsgefäße in s einer hierfür geeigneten Zentrifuge. So kann in einer bevorzugten Ausführungsform des erfindungsgemäßen Verfahrens mit der kommerziell erhältlichen DiaMed ID-Zentrifuge 12 S II mit den fest eingestellten Parametern (t = 10 min, v = 85 g) gearbeitet werden.

In einer weiteren bevorzugten Ausführungsform der vorliegenden Erfindung kann jedoch auch mit modifizierten Zentrifugations-verfahren gearbeitet werden, welche den Kinetiken der zugrundeliegenden Reaktionen besonders Rechnung tragen. Dies spiegelt sich in einer Reaktionsverstärkung vor allem schwacher Reaktionen wieder.

Weiterhin ist es für bestimmte Ausführungsformen des erfindungsgemäßen Verfahrens bevorzugt, mit Matrizes zu arbeiten, die einen zweiten Antikörper, beispielsweise einen sogenannten gruppenspezifischen Antikörper, wie etwa Anti-IgG, enthalten. Hierbei findet in den Reaktionsgefäßen eine zweiphasige Reaktion statt. Die erste Reaktion erfolgt in der Reaktionskammer, wenn in einer positiven Reaktion das Ligandenmolekül auf den synthetischen Partikeln, z.B. ein Antigen, mit dem Analyten, z.B. einem Antigen-spezifischen Antikörper, in der Probe reagiert. Es kann hier bereits zu Quervernetzungen zwischen verschiedenen synthetischen Partikeln kommen (Agglutination), was aber im Falle von IgG-Reaktionen oft nur zu sehr schwachen Agglutinaten führt. Die in der Matrix vorhandenen Zweitantikörper sorgen durch weitere Quervernetzung bereits gebildeter Agglutinationskomplexe für den erwünschten Effekt einer spezifischen Reaktionsverstärkung.

Bei einer Gesamttestdauer von nur 20 min. erlaubt das erfindungsgemäß beschriebene Testsystem eine signifikante Zeitersparnis gegenüber bekannten Systemen. Im Vergleich zu Testsystemen, die auf fixierten oder unfixierten Erythrozyten als Agglutinationsreagenz basieren, besteht ein zusätzlicher Vorteil in der weitaus längeren Haltbarkeit der synthetischen Partikel sowie der besseren Uniformität des Ausgangsmaterials.

Bei entsprechender Vorbereitung des Testsystems ist die Durchführung einfach und die Resultate können eindeutig abgelesen werden, so daß der Test auch von medizinischen Hilfspersonen durchgeführt werden kann. Die Anwendung ist denkbar einfach, da sie nur zwei Pipettierschritte umfaßt. Mit einer Akku-betriebenen Zentrifuge und einem ebensolchen Vortex-Gerät sind die Bestimmungen darüberhinaus leicht außerhalb fester Laboreinrichtungen ohne Stromanschluß durchführbar. Durch die geringe Menge an Probe, das Fehlen von Waschschritten sowie die Möglichkeit, die Reaktionsgefäße nach Benutzung zu verschließen, ist für kürzestmögliche Exposition des Personals mit potentiell infektiösem Material und größtmögliche Kontrolle von potentiell infektiösem Abfall gesorgt.

Ein weiterer Gegenstand der vorliegenden Erfindung sind synthetische Partikel mit einem mittleren Durchmesser ≤ 5 *µ*m, vorzugsweise von 1-5 *µ*m, besonders bevorzugt von 2 - 4 *µ*m und einer spezifischen Dichte ≥ 1,1 g/cm³, vorzugsweise 1,1 - 1,8 g/cm³, besonders bevorzugt 1,15 - 1,4 g/cm³, auf deren Oberfläche Ligandenmoleküle beispielsweise durch kovalente, adsorptive oder hochaffine Wechselwirkungen immobilisiert sind. Günstigerweise sind die Partikel gefärbt. Diese Partikel können als Agglutinationsreagenzien in einem Agglutinationstest, vorzugsweise in einem Gel-Agglutinationstest, eingesetzt werden.

Noch ein weiterer Gegenstand der vorliegenden Erfindung ist ein Reagenzienkit zum Nachweis eines Analyten in einer Probeflüssigkeit, umfassend
a) mindestens ein Reaktionsgefäß, das eine inerte Matrix, vorzugsweise eine partikuläre Matrix, enthält und
b) mindestens eine Art von synthetischen Partikeln, auf deren Oberfläche Ligandenmoleküle immobilisiert sind.

Bei bestimmten Arten des erfindungsgemäßen Bestimmungsverfahrens ist es bevorzugt, dem Reaktionsgemisch ein Detergens zuzusetzen. Beispiele für geeignete Detergenzien sind ionische Detergenzien, wie SDS, oder nichtionische Detergenzien, wie Triton X-100 und Tween 20. Weiterhin kann auch der Zusatz von Mucinen bevorzugt sein. Durch Zusatz von Detergenzien oder/und Mucinen kann die Uniformität der Partikel in der Suspension bzw. während der Gelpassage verbessert werden. Dies führt zu einer vollständigeren Sedimentierung bei negativer Reaktion.

Ebenfalls kann es sich als zweckmäßig erweisen, die Reaktion unter Zusatz eines reduzierenden Agens, z.B. eines Sulfhydrylreagenz, wie 2-Mercapto-Ethanol, Dithiothreitol oder Natriumdithionit, oder eines anderen reduzierenden Agens, wie etwa Tributylphosphin, durchzuführen. Durch reduzierende Agenzien können unerwünschte unspezifische Reaktionen, wie sie z. B. durch IgM-Antikörper hervorgerufen werden, verringert werden.

Weiterhin kann bei bestimmten Tests durch eine Modifizierung der Zentrifugationsbedingungen eine Verbesserung der Testergebnisse erreicht werden. Dies gilt insbesondere bei Tests, bei denen eine mit einem Rezeptor, z. B. einem Zweitantikörper beschichtete Matrix verwendet wird. Eine bevorzugte Modifizierung der Zentrifugationsbedingungen umfasst eine Intervallzentrifugation, d. h. einen ersten Zentrifugationsschritt, dann eine Pause und dann einen zweiten Zentrifugationsschritt. Gegebenenfalls kann die Zentrifugation in mehreren Intervallschritten durchgeführt werden. Der erste Zentrifugationsschritt ist vorzugsweise nur ein kurzer Zentrifugationsschritt mit einer Dauer von bis zu 1 min, vorzugsweise bis zu 30 s. Dann erfolgt eine Pause, die beispielsweise 1-10 min, bzw. ca. 5 min betragen kann. Anschließend erfolgt ein zweiter Zentrifugationsschritt von längerer Dauer und gegebenenfalls auch höherer g-Zahl.

Anhand der nachfolgenden Beispiele und Figuren werden bestimmte Aspekte der Erfindung näher erläutert. Es zeigen:

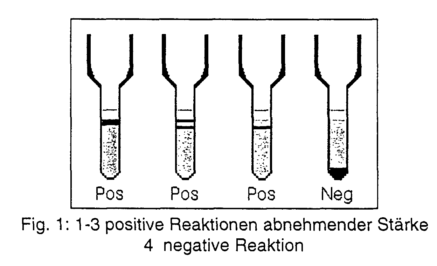

- Fig. 1 :: die bei positiver, schwach positiver und negativer Reaktion im erfindungsgemäßen Verfahren auftretenden Resultate,

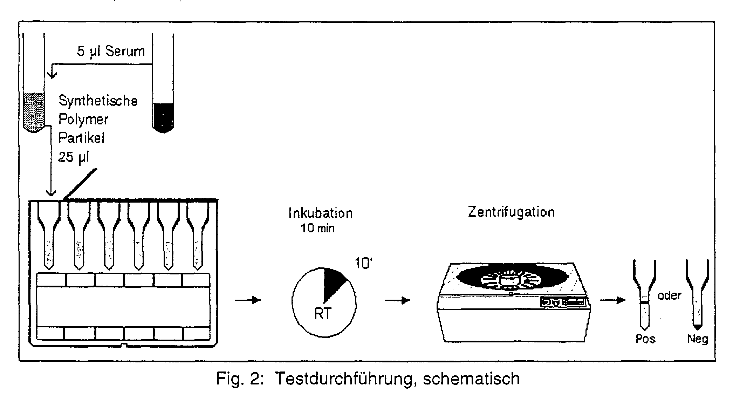

- Fig. 2:: eine schematische Darstellung der Testdurchführung,

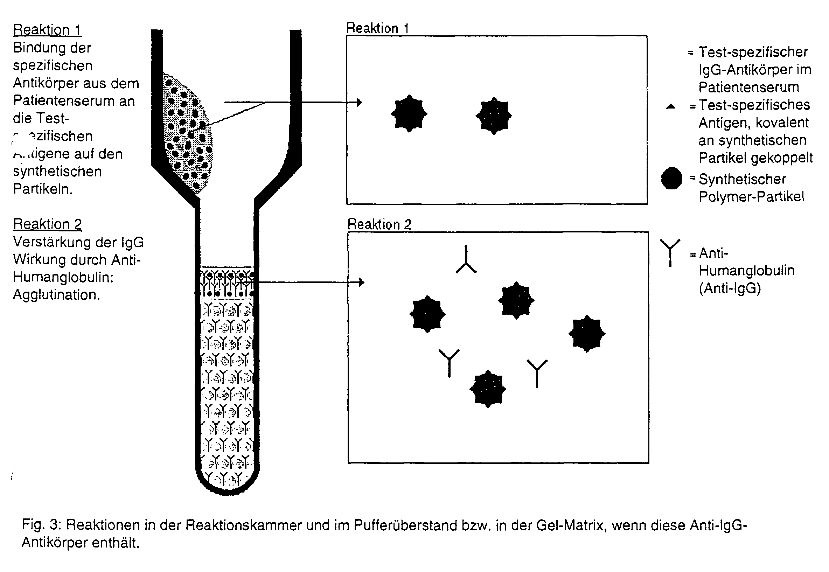

- Fig. 3 :: die Reaktionen, die in einem Mikroreaktionsgefäß stattfinden, bei dem die Matrix Anti-IgG-Antikörper enthält:
a) in der Reaktionskammer und
b) im Pufferüberstand der Matrix bzw. in der Matrix,

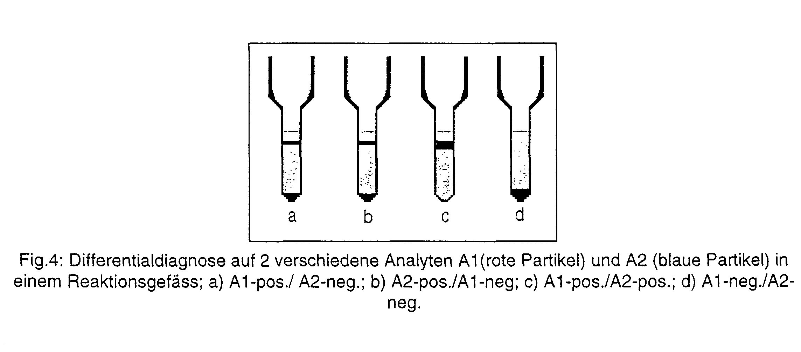

- Fig. 4 :: das schematische Ergebnis einer Differentialdiagnose auf zwei verschiedene Analyten (A1 und A2) in einem Reaktionsgefäß:
a) Patientenprobe 1, Antikörper positiv für A1;
b) Patientenprobe 2, Antikörper positiv für A2;
c) Probe 3 (Mischung der Seren von Patienten 1 und 2), Antikörper positiv für A1 und A2;
d) Patientenprobe 4, Antikörper negativ für A1 und A2 und

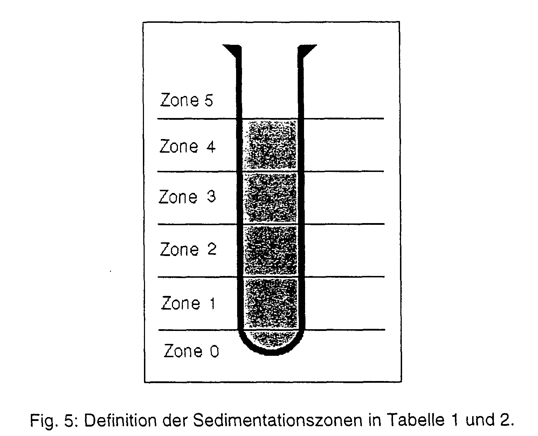

- Fig. 5:: die schematische Darstellung der Reaktionszonen, auf die in Tabelle 1 Bezug genommen wird. Zone 0 bedeutet: die Partikel bilden nach Zentrifugation ein Sediment aus, wie es für eine negative Reaktion wünschenswert ist. Zone 5 bedeutet: Keine Durchdringung der Gel-Matrix

### Beispiele:

### 1. Versuche mit synthetischen Partikeln des Standes der Technik, die im Gel-Test nicht funktionieren (Vergleich)

### Testdurchführung:

Die Suspensionen kommerziell erhältlicher, farbiger synthetischer Partikel, die üblicherweise in Konzentrationen von 10 % feste Partikel (w/v) vorliegen, wurden mit einer 10 mM PBS-Lösung, pH 7.4 (Sigma P 4417), 0.1 % Tween-20 (Sigma P 7949) (v/v) gewaschen und in derselben Lösung auf eine Konzentration von 0.15 % feste Partikel eingestellt.

Von dieser Suspension wurden jeweils 25 *µ*l in ein Mikroreaktionsröhrchen pipettiert und unter den in Tabelle 1 angegebenen Bedingungen sedimentiert.

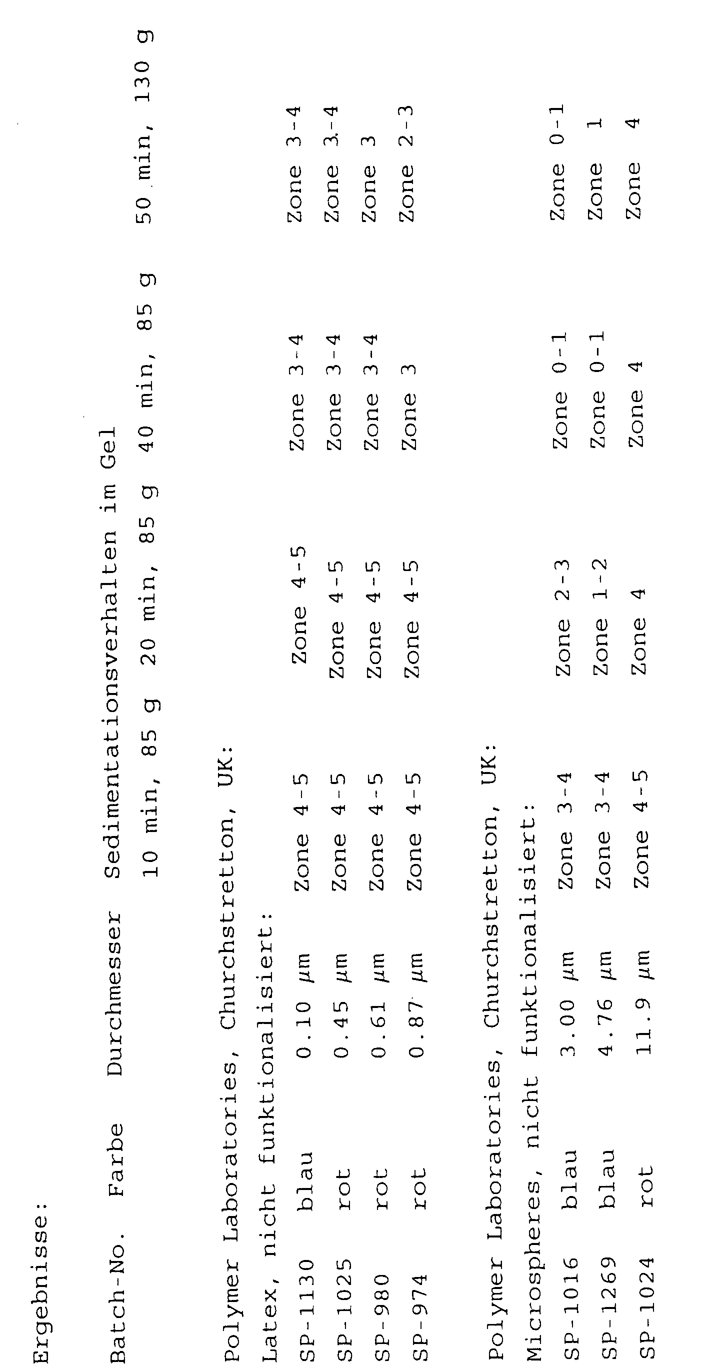

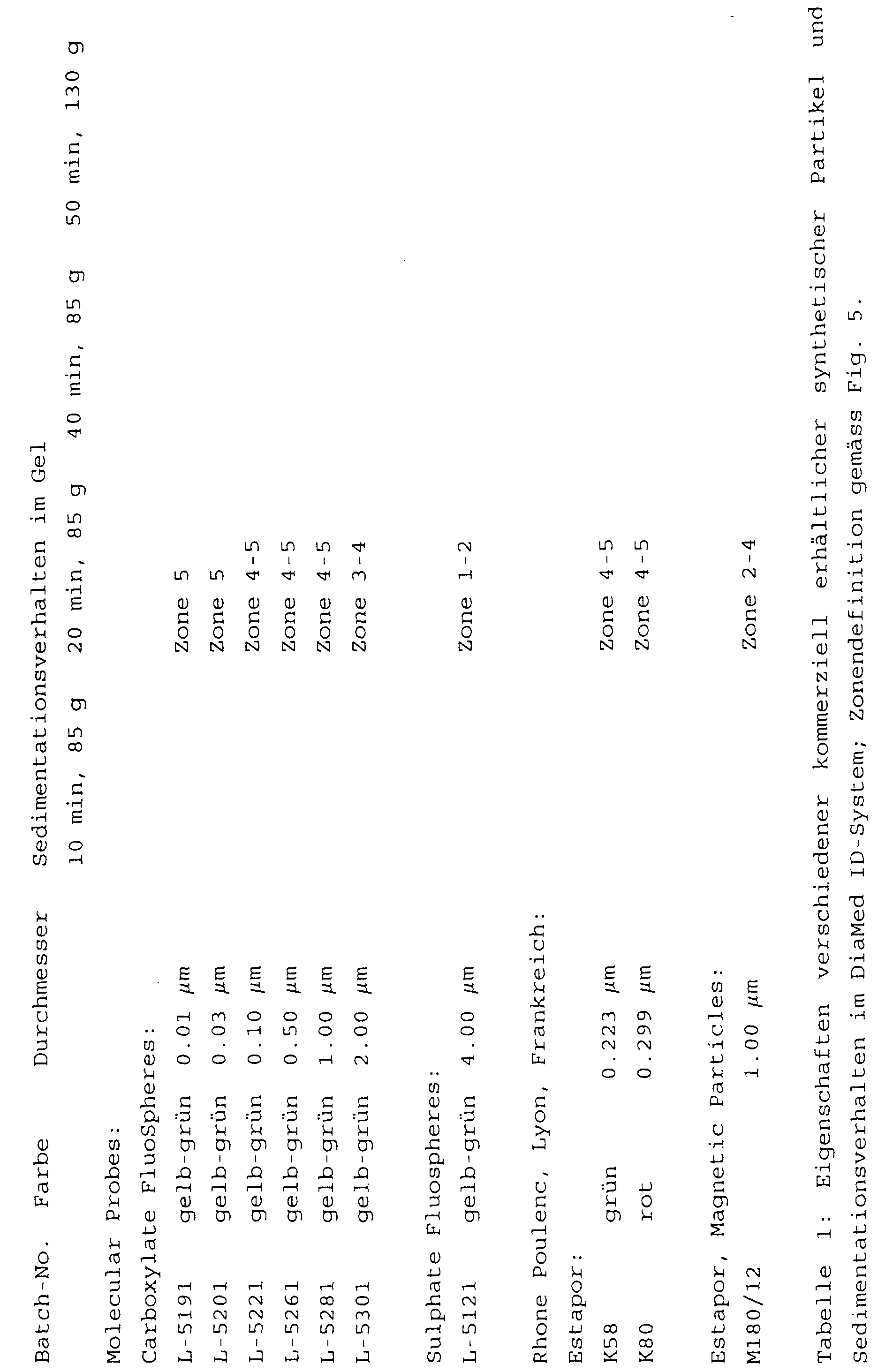

### 2. Herstellung geeigneter synthetischer Partikel

Die in den folgenden Beispielen eingesetzten synthetischen Partikel wurden mit Dispersions-Polymerisation hergestellt, da diese Methode im allgemeinen zur Herstellung von Partikeln im Bereich von 1 bis 5 *µ*m am besten geeignet ist. In einem ersten Schritt wurden Basis-Partikel aus Polystyrol synthetisiert (2a). Danach wurden die Basis-Partikel durch eine Copolymerisierung mit Styrol/Bromstyrol zu High-Density Partikeln geschwollen (2b). In dem darauffolgenden dritten Schritt erfolgte die Einfärbung (2c).

### 2a. Polymerisierung der Basis-Partikel

### Durchführung:

60 g 1-Hexadecanol (Aldrich 25,874-1), 216 g Polyvinylpyrrolidon 40000 (PVP) (Aldrich 85,656-8), 1744 g dest. Styrol (Aldrich S497-2) und 10261 g Industrie-Spiritus (Banners IMS 99) wurden in einen 20 1 Rundkolben-Reaktor (ausgerüstet mit Rührer, Wasser-Kondensator und Stickstoff-Begasung) eingewogen. Die Rührgeschwindigkeit wurde auf 35 - 40 U/min eingestellt. Die Mischung wurde für 16 Stunden bei Raumtemperatur begast. Darauf wurde die Temperatur auf 70 ° C erhöht. 17.4 g Azo-bisisobutyronitril (Fisons A/9050/50) wurden nun dem Reaktionsgemisch zugefügt, wobei sich eine weisse Emulsion ausbildete. Nach weiteren 24 Stunden wurde das Reaktionsgemisch auf Raumtemperatur abgekühlt. Die resultierenden Partikel wurden durch wiederholte Zentrifugation in Methanol gewaschen (3000 rpm, 5 min) und nach dem letzten Waschschritt in einer 0.1 % % (w/v) SDS (Fisons S/5200/53) -Lösung resuspendiert. Aus diesem Ansatz gingen 1573 g monodisperse Polystyrol-Partikel mit 2.3 *µ*m Durchmesser hervor.

### 2b. Herstellung von "High Density" Partikeln

### Durchführung:

118.3 g einer 10 % % (w/v) Suspension von 2.3 *µ*m Partikeln (hergestellt gemäss Beispiel 2a) und 250 g einer Polyvinylalkohol (Harco 26-88) (PVA) (5 % w/v)-Lösung wurden in einen 1 1 Rundkolben-Reaktor (ausgerüstet mit Rührer, Wasser-Kondensator und Stickstoff-Begasung) eingewogen und die Rührgeschwindigkeit auf 250 U/min eingestellt. 1.5 g Benzoyl-Peroxid (Aldrich 22,887-7) wurde in 21.6 g 4-Bromstyrol (Avocado 17896) aufgelöst. Diese Lösung wurde in 250 ml einer 0.5 % (w/v) SDS-Lösung emulgiert. Die Bromstyrol Emulsion wurde dann dem Rundkolben-Reaktor zugegeben. Nach 3 Stunden wurden 125 g einer 1 % (w/v) Na-Dichromat (Fisons S/3560/60) Lösung zu dem Reaktor zugegeben und die Temperatur auf 70°C erhöht. Nach weiteren 16 Stunden wurde die Temperatur auf 80°C erhöht und für weitere 3 Stunden weiterreagiert. Danach wurde das Reaktionsgemisch auf Raumtemperatur abgekühlt. Die resultierenden Partikel wurden durch wiederholte Zentrifugation in Wasser gewaschen und ergaben eine Ausbeute von 26.98 g Partikel von nunmehr einem Durchmesser von 3.1 *µ*m. Der nominelle Gehalt an polymerem Bromstyrol dieser Partikel betrug 65 % (w/v). Die Sedimentationsrate einer 1 % (w/v) Suspension dieser Partikel in Wasser betrug 5.7 mm/hr, die theoretische Dichte liegt bei 1.28 g/cm³ (s. Tabelle 2).

Nach diesem Verfahren wurden verschiedene Partikel gleichen Durchmessers, aber mit unterschiedlichen Verhältnissen Bromstyrol/Styrol und damit unterschiedlich hoher Dichte polymerisiert (Tabelle 2).

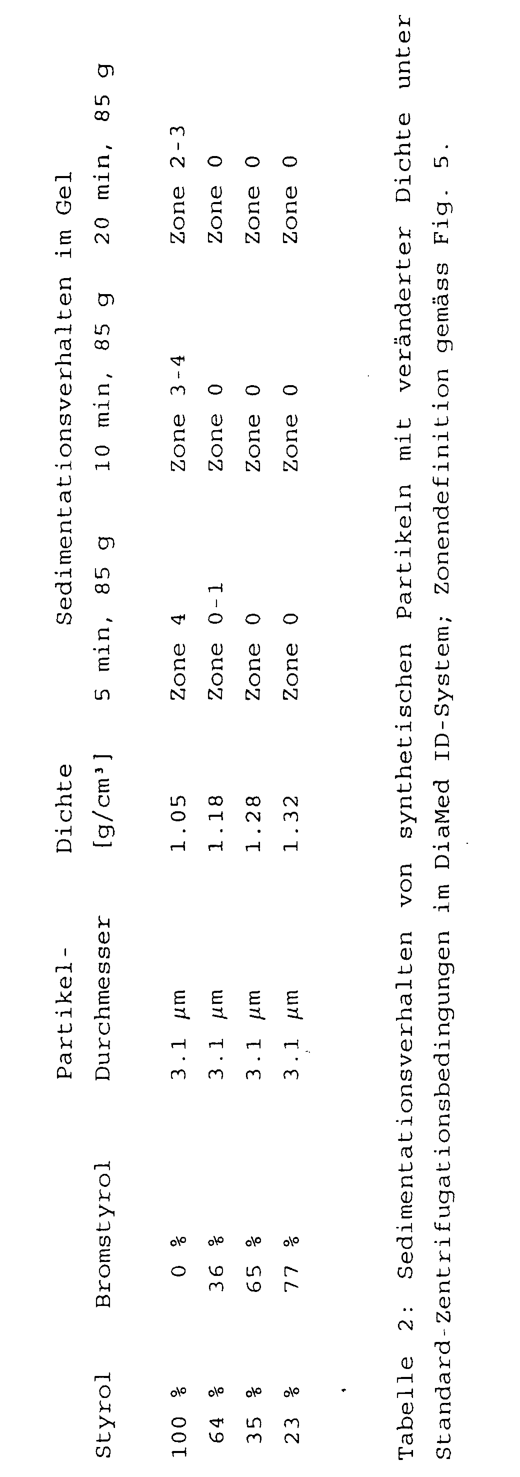

### 2c. Einfärbung der High Density Partikel

### Durchführung:

Sudan IV (rot) :
   200 g einer 10 % (w/v) Suspension synthetischer High density Partikel (hergestellt wie in 2a, 2b beschrieben) mit 3.1 *µ* m Durchmesser, 11.6 g einer 5 % (w/v) Lösung Polyvinylalkohol (Harco 26-88), 40 g Methanol (Hammond) und 240 g Wasser wurden in einen 1 l Rundkolben-Reaktor (ausgerüstet mit PTFE-Rührer, Wasser-Kondensator) eingewogen. 0.4 g Farbstoff Sudan IV (Kodak 112 6150) wurden in 30 g Dichlormethan (Fisons D/1852/25) gelöst und die Lösung in 250 ml einer 0.5 % (w/v) SDS-Lösung emulgiert. Die Dichlormethan-Emulsion wurde dann bei Raumtemperatur in den Reaktor gegeben. Nach 4 Stunden wurde die resultierende Emulsion roter Partikel in ein 2 l Becherglas gegeben. Um das Dichlormethan möglichst schnell zu evaporieren, wurde die Oberfläche der Suspension unter Rühren mit Stickstoff begast. Zur Maximierung der Evaporation wurde die Suspension zusätzlich leicht erwärmt. Die resultierenden roten Partikel wurden durch wiederholte Zentrifugation in Wasser gewaschen (1000 rpm, 5 min). Sie ergaben eine Ausbeute von 19.8 g.
Ferro FW 1263 (blau):
   50 g einer 10 % (w/v) Suspension synthetischer High density Partikel (hergestellt wie in 2a, 2b beschrieben) mit 3.1 Um Durchmesser, 2.9 g einer 5 % (w/v) Lösung Polyvinylalkohol (Harco 26-88) und 60 g Wasser wurden in einen 1 l Rundkolben-Reaktor (ausgerüstet mit PTFE-Rührer, Wasser-Kondensator) eingewogen. 0.184 g Farbstoff Ferro FW1263 (Ferro Normandy Plastics Ltd, Westgate, Aldridge, GB) wurden in 7.5 g Dichlormethan (Fisons D/1852/25) gelöst und diese Lösung in 62.5 ml einer 0.5 % (w/v) SDS-Lösung emulgiert. Die Dichlormethan-Emulsion wurde dann bei Raumtemperatur in den Reaktor gegeben. Nach 2 Stunden wurde die resultierende Emulsion blauer Partikel in ein 500 ml Becherglas gegeben. Um das Dichlormethan möglichst schnell zu evaporieren, wurde die Oberfläche der

Suspension unter Rühren mit Stickstoff begast. Zur Maximierung der Evaporation wurde die Suspension zusätzlich leicht erwärmt. Die resultierenden blauen Partikel wurden durch wiederholte Zentrifugation in Wasser gewaschen (1000 rpm, 5 min). Sie ergaben eine Ausbeute von 4.8 g.

### 3. Funktionalisierung und Streptavidinisierung der Partikel-Oberfläche

a) Herstellung von Aldehyd-Partikeln
   Ein 1 ml Aliquot roter 3.1 *µ*m High density Partikel (17.1 %) (w/v) (hergestellt wie in Beispiel 2 a bis c beschrieben) wurde in ein 15 ml Zentrifugenröhrchen gegeben und 3 mal durch Zentrifugation mit ultrareinem (18 MegOhm) Wasser gewaschen (1000 rpm, 5 min). Zu den so gewaschenen Partikeln wurden 15 ml einer Lösung von Rinderserumalbumin (2.67 mg/ml) (Sigma A9647) in 10 mM PBS, pH 7.4, 0.05 % NaN₃ zugegeben und 4 Stunden bei Raumtemperatur gerührt. Die Partikel wurden 4 mal durch Zentrifugation mit 15 ml Wasser gewaschen (1000 rpm, 5 min). Darauf wurden die Partikel in 10 ml Wasser resuspendiert, um eine Konzentration von 1.7 % (w/v) zu ergeben. Zu einem 0.5 ml Aliquot dieser Suspension wurden 3.75 ml Wasser zugegeben, um eine 0.2 % (w/v) Suspension zu erhalten. Hierzu wurden 4.25 ml einer 2 % % Glutaraldehyd-Lösung in Wasser zugegeben und das Reaktionsgemisch für eine Stunde bei Raumtemperatur gerührt. Die Partikel wurden 4 mal durch Zentrifugation mit 15 ml Wasser gewaschen (1000 rpm, 5 min).
b) Herstellung von Streptavidin-Partikeln
   Eine Suspension von 8 mg roten 3.1 *µ*m High density Partikeln (hergestellt wie in 2 a-c beschrieben und funktionalisiert wie in 3a beschrieben) wurde in 4 ml Wasser resuspendiert und für 1 min im Ultraschall-Wasserbad (Sonomatic von Langford Ultrasonics, Birmingham, GB) inkubiert. Darauf wurden 4 ml eines 50 mM Natriumacetat-Puffers, pH 3.25, zugegeben, gefolgt von 1 ml einer wässrigen Streptavidin-Lösung (4 mg/ml). Nach weiteren 2 min im Ultraschall-Wasserbad wurde sofort 1 ml einer 50 mM Natriumcyanborhydrid (Aldrich 15,615-9)-Lösung in Acetatpuffer zugegeben. Dieses Gemisch wurde unter leichtem Schütteln über Nacht bei Raumtemperatur inkubiert. Die Partikel wurden 3 mal durch Zentrifugation mit 15 ml Wasser gewaschen (1000 rpm, 5 min) und in 5.33 ml 10 mM PBS, pH 7.4, resuspendiert, so dass die Partikel-Konzentration auf 0.15 % (w/v) eingestellt wurde.
c) Herstellung von Polylysin-Partikeln
   Zu einer Suspension von 500 mg roten 3.1 *µ*m High density Partikeln in 1 ml Wasser (hergestellt wie in 2a-c und funktionalisiert wie in 3a beschrieben) wurden 4 ml 50 mM Na-Acetat, pH 3.25 gegeben. Dann wurden 4 ml einer 1 mg/ml Poly-L-Lysin (Sigma P 2636) -Lösung in Wasser zugegeben. Das Reaktionsgemisch wurde für 4 min im Ultraschall-Wasserbad inkubiert, bevor 1 ml 50 mM Natriumcyanborhydrid in Acetat-Puffer zugegeben wurde. Das Reaktionsgemisch wurde unter leichtem Schütteln über Nacht bei Raumtemperatur inkubiert. Die Partikel wurden 3 mal durch Zentrifugation mit Wasser gewaschen (1000 rpm, 5 min) und in 333 ml 10 mM PBS, pH 7.4, resuspendiert, so dass die Partikel-Konzentration in der Suspension auf 0.15 % (w/v) eingestellt wurde.

### 4. Kopplung von Liganden an die funktionalisierten Partikel

a) Kovalente Kopplung
   0.1 ml Lösungen von biotinylierten Antigenen wurden zu 1 ml einer 0.15 % % (w/v) Suspension roter 3.1 *µ*m High density Partikel (hergestellt wie in 2 a - c, funktionalisiert und streptavidinisiert wie in Beispiel 3a, 3b beschrieben) in ein Eppendorf-Mikroröhrchen gegeben und für 30 min unter Schütteln inkubiert. Ungebundenes Antigen wurde durch zweimalige Zentrifugation (1000 rpm, 5 min) und Resuspension mit 1 ml des obengenannten Puffers weggewaschen, so dass die Endkonzentration der Partikelsuspension wieder 0.15 % (w/v) betrug.
b) Nichtkovalente Kopplung
   Zu 500 *µ*l einer 0.15 % Suspension roter 3.1 *µ*m High density Polylysin-Partikel (hergestellt wie in 2 a-c und 3a, 3c beschrieben) wurden 10 *µ*l einer 20 *µ*g/ml ds DNA (Sigma D 1501)-Lösung in 10 mM PBS, pH 7.4, zugegeben. Nach einem Vortex-Schritt (Bender & Hobein, Vortex Genie 2) für 5 sec auf Stufe 8 wurde für 12 Stunden unter leichtem Schütteln bei Raumtemperatur inkubiert. Die Suspension wurde 2 mal durch Zentrifugation mit 1 ml 10 mM PBS, pH 7.4, gewaschen (1000 rpm, 5 min) und erneut in 500 *µ*l 10 mM PBS, pH 7.4, resuspendiert.

### 5. Pufferzusammensetzung der Gelmatrix

Die Gelmatrix in den Mikroreaktionsgefässen befand sich in folgendem wässrigen Puffermilieu: 5 mM KH₂PO₄ (Merck 4873) /Na₂HPO₄ (Merck 6580), 150 mM NaCl (Fluka 71381), 0.024 % (w/v) NaN₃ (Fluka 71290), 1.875 % (v/v) Albumin (Miles 81-177), 0.05 % (w/v) EDTA (Fluka 03685), 1.3 mM Tris (Merck 8382), 1.25 mM N-Acetyl-L-Cystein (Merck 12422), 0.025 % % (w/v) Mucin (Sigma M 1778). Weiterhin enthielt die Gelmatrix gegebenenfalls testabhängig unterschiedliche Mengen an Anti-Humanglobulin.

### 6. Chagas-Antikörper Test (Kovalente Bindung von synthetischen Peptiden)

a) Antigene:
   Synthetische Peptide Ag-2, TcD und TcE stammen von Alta Bioscience (Birmingham, UK).
b) Kopplung:
   Je 1 ml einer 0.15 % (w/v) Suspension roter 3.1 *µ*m High density Partikel (hergestellt wie in 2 a - c, funktionalisiert und streptavidinisiert wie in Beispiel 3a, 3b beschrieben), wurden mit 2 ng TcD, 35 ng Ag-2 und 282 ng TcE gekoppelt, welche nach Standardtechniken mit einem biotinyliertem Lysin zusätzlich zur Antigen-Sequenz synthetisiert und über RP-HPLC ebenfalls nach Standardmethoden auf > 90 % Reinheit gereinigt wurden. Hierfür wurde von den Peptiden eine 10 x Stammlösung in H₂O (20 ng TcD, 350 ng Ag-2, 2820 ng TcE pro ml) bereitet. Für die Kopplung wurden dann jeweils 1 Vol. Peptidlösung zu 10 Vol. einer 0.15 % (w/v) Suspension synthetischer Partikel zugegeben und schnell vermischt. Das Reaktionsgemisch wurde dann für 30 min bei Raumtemperatur unter leichtem Schütteln inkubiert. Danach wurde die Partikel-Suspension für 5 min bei 1000 rpm zentrifugiert, der Überstand dekantiert und zweimal mit 10 mM PBS, pH 7.4, gewaschen. Die finale Konzentration der Partikel betrug in der Suspension wieder 0.15 % (w/v). Die so sensitivierten Partikel wurden bei 4 ° C gelagert.
c) Testdurchführung:
   Eine 0.15 % (w/v) Suspension der wie in 4a beschrieben sensitivierten synthetischen Partikel wurde für 5 min im Ultraschall-Wasserbad behandelt. Dann wurden je 25 *µ*l in eine Karte mit 6 mit einer Anti-Humanglobulin haltigen (0.5 %, v/v) Gel-Suspension vorgefüllten Mikroröhrchen (s. Beispiel 5: ID-Karte, DiaMed, Cressier sur Morat) gegeben. Anschliessend wurden 1.5 *µ*l Patientenserum oder -Plasma zugegeben. Nach 10 min Inkubation bei Raumtemperatur wurde für 10 min bei 85 g in einer speziell für die Mikroröhrchen entwickelten Zentrifuge (ID-Centrifuge 12 S II, DiaMed, Cressier sur Morat) zentrifugiert. Die Ergebnisse können sofort nach der Zentrifugation ausgewertet werden.
d) Auswertung:
   Positive Resultate sind als deutliche Bande auf dem Gel oder 1 bis zwei mm in das Gel eingedrungen detektierbar. Auch über das ganze Gel verteilte Agglutinate zeigen positive Reaktionen an. Negative Resultate sind an einem deutlichen Sediment der auf den Boden des Gefässes sedimentierten synthetischen Partikel zu erkennen, eine Bande im oder auf dem Gel gibt es in diesem Falle nicht.

### 7. Visceral Leishmaniasis Antikörper Test (Kovalente Bindung von rekombinantem Antigen)

a) Antigene:
   Rekombinantes Antigen rK39 von Corixa Inc., Seattle, USA.
b) Biotinylierung und Kopplung:
   1 ml rekombinantes Antigen rK39 (0.6 mg/ml) in 10 mM Tris, pH 8.0, wurde gegen 10 mM PBS, pH 7.4 (Sigma P 4417) bei 4°C dialysiert und dann durch ein Puradisc 25 AS Membranfilter (Whatman) filtriert. Die Biotinylierung erfolgte mit Sulfo-NHS-Biotin (Pierce 21217). Ein 500 *µ* l Aliquot des rK39 in 10 mM PBS, pH 7.4 (300 *µ*g) wurde mit 17.5 *µ*l einer wässrigen 1 mg/ml Lösung Sulfo-NHS-Biotin versetzt. Das Reaktionsgemisch wurde für 150 min bei Raumtemperatur inkubiert. Das biotinylierte rK39 wurde nun mit einer KwikSep-Entsalzungssäule (Pierce) von freiem Biotin getrennt. Die Elution erfolgte mit 10 mM PBS, pH 7.4, 0.05 % NaN3.
   Je 1 ml einer 0.15 % (w/v) Suspension roter 3.1 *µ*m High density Partikel (hergestellt wie in 2 a - c, funktionalisiert und streptavidinisiert wie in Beispiel 3a, 3b beschrieben) wurden dann mit 160 ng biotinyliertem rK39 gekoppelt. Hierfür wurde eine 10 x Stammlösung in H₂O (1600 ng rK39 pro ml) hergestellt. Die eigentliche Kopplung erfolgte dann wie in Beispiel 6b beschrieben.
c) Testdurchführung:
   Die Testdurchführung ist derjenigen in Beispiel 6 identisch, nur dass im vorliegenden Fall mit einer Suspension von synthetischen Partikeln gearbeitet wird, welche mit rK39 sensitiviert wurde.
d) Auswertung: wie in Beispiel 6.

### 8. Hepatitis B surface Antigen Test (Kovalente Bindung von monoklonalen Antikörpern)

a) Antikörper:
   Der gegen das Hepatitis B surface Antigen gerichtete monoklonale Antikörper (MAb) MIH9701 detektiert beide Subtypen ad und ay und stammte von Medix Biotech, Walchwil, Schweiz.
b) Biotinylierung und Kopplung:
   200 *µ*l einer 3 mg/ml Lösung MIH9701 wurden mit 0.1 M Natriumacetat-Puffer, pH 5.5, auf 1 ml verdünnt und gegen denselben Puffer über Nacht bei 4°C dialysiert. Zu einem 900 *µ*l Aliquot der resultierenden MAb Lösung wurden 900 *µ*l einer eiskalten 20 mM Natriummetaperiodat-Lösung in Wasser zugegeben und das Reaktionsgemisch für 20 min bei 0°C im Dunkeln inkubiert. Die Reaktion wurde durch Zugabe von 11 *µ*l einer 10 % (w/v) Glyzerin-Lösung gestoppt. Die MAb wurden von den übrigen Reaktionskomponenten getrennt durch einen Chromatographie-Schritt über eine Kwiksep Entsalzungs-Säule. Dabei wurde mit 0.1 M Acetat-Puffer, pH 5.5 eluiert. Das Volumen der MAb nach Elution war 2.7 ml. Hierzu wurden 270 *µ* l einer 50 mM Lösung von EZ-Link Biotin-LC-Hydrazid (Pierce 21340) in Dimethylsulfoxid (Sigma D 8418) zugegeben und unter leichtem Schütteln für 2 Stunden bei Raumtemperatur inkubiert. Das Reaktionsgemisch wurde nun bei 4°C gegen 10 mM PBS, pH 7,4, 0.05 % NaN₃ dialysiert und darauf durch ein 0.2 *µ*m Puradisc AS Filter (Whatman) filtriert. Mit einer Kwiksep-Entsalzungssäule wurde ein abschliessender Entsalzungsschritt wiederum gegen 10 mM PBS, pH 7.4, 0.05 % NaN₃ durchgeführt.
   Die so biotinylierten MAb wurden in demselben PBS-Puffer auf 35 *µ*g/ml eingestellt. Zu 1 ml einer 0.15 % (w/v) Suspension von roten 3.1 *µ*m High density Partikeln (hergestellt wie in 2a-c, funktionalisiert und streptavidinisiert wie in 3a, 3b beschrieben) wurden nun 100 *µ*l der verdünnten MAb-Lösung zugegeben. Dieses Reaktionsgemisch wurde für 30 min bei Raumtemperatur unter leichtem Schütteln inkubiert. Die Partikel wurden dann 3 mal durch Zentrifugation mit demselben Puffer gewaschen (1000 rpm, 5 min).
c) Testdurchführung
   Die Testdurchführung war wie in Beispiel 6 mit folgenden Unterschieden:
   i) Es wurde mit einer Suspension von synthetischen Partikeln gearbeitet, welche mit MAb MIH9701 sensitiviert wurde.
   ii) Die Gel-Matrix enthielt keine Anti-Humanglobuline
d) Auswertung: wie in Beispiel 6.

### 9. Test auf anti-dsDNA Antikörper (Nicht-kovalente Bindung von ds-DNA)

a) Antigen:
   doppelsträngige (ds)-DNA, Type I stammte von Sigma (D-1501).
b) Kopplung:
   Zu 500 *µ*l einer 0.15 % (w/v) Suspension roter 3.1 *µ*m High density Partikel (hergestellt wie in 2a-c und 3a, 3c) wurden 10 *µ*l einer 20 *µ*g/ml dsDNA (Sigma D 1501) in 10 mM PBS, pH 7.4, zugegeben. Nach einem Vortex-Schritt (Bender & Hobein, Vortex Genie 2) für 5 sec auf Stufe 8 wurde unter leichtem Schütteln für 12 Stunden bei Raumtemperatur inkubiert. Die Suspension wurde 2 mal durch Zentrifugation mit 1 ml 10 mM PBS, pH 7.4, gewaschen und in 500 *µ*l 10 mM PBS, pH 7.4, resuspendiert. Die finale Konzentration der Partikel betrug wieder 0.15 % (w/v), die sensitivierten Partikel wurden bei 4°C aufbewahrt.
c) Testdurchführung: Wie in Beispiel 6.
d) Auswertung: Wie in Beispiel 6.

### 10. HIV1/HIV2 Antikörper Differenzierungs-Test (Anwendung zweier verschiedenartig sensitivierter Spezies synthetischer Partikel mit unterschiedlichen Farben, aber sonst gleichen physikalischen Eigenschaften)

a) Antigene:
   Synthetische Peptide -Sequenzen aus HIV-1 gp41 und HIV-2 gp36-von Alta Bioscience (Birmingham, UK).
b) Kopplung wie in Beispiel 6 mit 72 ng gp-41 Peptid und 40 ng gp36 Peptid je 1 ml einer 0.15 % (w/v) Partikel-Suspension. Es wurden rote Partikel (hergestellt wie in 2a-c, funktionalisiert und streptavidinisiert wie in Beispiel 3a, 3b beschrieben) mit dem HIV-2 spezifischen Peptid (Antigen-Sequenz aus gp36) und blaue Partikel. (hergestellt wie in 2a-c, funktionalisiert und streptavidinisiert wie in Beispiel 3a, 3b beschrieben) mit dem HIV-1 spezifischen Peptid (Antigensequenz aus gp41) sensitiviert.
c) Testdurchführung
   Wie in Beispiel 6 mit folgenden Unterschieden: Es wurden jeweils 10 *µ*l der Suspension roter und der Suspension blauer Partikel in die Reaktionskammer eines Mikroröhrchens pipettiert. Darauf wurden 2.5 *µ*l Patientenserum zugegeben.
d) Auswertung
   HIV-1/HIV-2 positive Resultate wurden als deutliche rot/blaue Bande auf dem Gel oder 1 bis zwei mm in das Gel eingedrungen detektierbar. Es gab in diesem Falle kein Sediment von Partikeln. HIV-1 Antikörper positive Resultate zeigten eine blaue Bande auf dem Gel oder ins Gel eingedrungen, während die roten Partikel ein Sediment auf dem Boden des Reaktionsgefässes ausbildeten. HIV-2 Antikörper positive Resultate zeigten ein umgekehrtes Bild. HIV-1/HIV-2 negative Resultate waren an dem deutlich rot-blauen Sediment auf dem Boden des Mikroreaktions-Gefässes zu erkennen.

### 11. Spezifische Detektion von Nicht-IgM-Antikörpern

a) Antigene:
   S. aureus Protein A stammte von Sigma (P 6650); Ziege anti-human IgM-Antikörper (*µ*-Ketten spezifisch)-Biotin Konjugat F (ab)₂ Fragment stammte von Sigma (B 2641).
b) Biotinylierung und Kopplung:
   Die Biotinylierung von Protein A erfolgte wie in Beispiel 8 beschrieben. Die biotinylierten anti-IgM-Antikörper wurden in 10 mM PBS, pH 7.4, in einem Ansatz auf 40 *µ*g/ml 3.1 *µ*m High density Partikel (1.5 %, w/v) eingestellt, in einem weiteren Ansatz auf 1 *µ*g/ml Partikel (0.15 % w/v), das biotinylierte Protein A auf eine Konzentration von 1 ug/ml Partikel (0.15 % w/v). Zu je 1 ml einer Suspension von roten 3.1 *µ*m High density Partikeln (hergestellt wie in 2a-c, funktionalisiert und streptavidinisiert wie in 3a, 3b beschrieben) wurden dabei 100 *µ*l jeweils 10 fach konzentrierter Lösungen mit biotinyliertem anti-IgM-Antikörper (400 *µ*g/ml bzw. 10 *µ*g/ml) bzw. Protein A (10 *µ*g/ml) zugegeben.
   Dieses Reaktionsgemisch wurde für 30 min bei Raumtemperatur unter leichtem Schütteln inkubiert. Die Partikel wurden darauf 3 mal durch Zentrifugation mit demselben Puffer gewaschen (1000 rpm, 5 min).
c) Testdurchführung
   1 ml der mit anti-IgM sensitivierten Partikel wurden für 5 min bei 1000 rpm zentrifugiert und der Überstand verworfen. Es wurden 20 *µ*l einer 1 zu 10 Verdünnung des Serums einer gesunden Person zugegeben. Nach 5 s Vortex auf Stufe 8 wurde das Reaktionsgemisch für 30 min bei Raumtemperatur unter leichtem Schütteln inkubiert, um die IgM Antikörper des Serums zu adsorbieren. Nach einer erneuten Zentrifugation für 5 min bei 1000 rpm wurde der Überstand abgenommen. In einer anti-IgG haltigen Karte (wie in Beispiel 6 beschrieben) wurden nun folgendermassen pipettiert:
   Position 1: 25 *µ*l Protein A Partikel (1 *µ*g/ml)
   Position 2: 25 *µ*l Protein A Partikel (1 *µ*g/ml)
   Position 3: 25 *µ*l anti-IgM Partikel (1 *µ*g/ml)
   Position 4: 25 *µ*l anti-IgM Partikel (1 *µ*g/ml).
   Zu Position 1 und 3 wurden jeweils 5 *µ*l des 1 zu 10 verdünnten Serums vor Adsorption, in Position 2 und 4 je 5 *µ*l des 1 zu 10 verdünnten Serums nach Adsorption gegeben. Nach 10 min Inkubation bei Raumtemperatur wurde für 10 min bei 85 g in einer speziell für die Mikroröhrchen entwickelten Zentrifuge (ID-Centrifuge 12 S II, DiaMed, Cressier sur Morat) zentrifugiert.
d) Auswertung:
   Beide Kontroll-Röhrchen 1 und 3 zeigten deutlich positive Reaktionen wie in Beispiel 6 beschrieben. Von den Testansätzen 2 und 4 zeigte 2 eine positive Reaktion, die sich nicht von der in Röhrchen 1 unterschied, während in Position 4 die Partikel ein Sediment auf dem Boden des Reaktionsgefässes ausbildeten und damit eine negative Reaktion anzeigten. Das heisst, in diesem Test konnte spezifisch eine Nicht-IgM-Anti-körper-Reaktion nachgewiesen werden.

### 12. Modifizierte Zentrifugation für synthetische Partikel

a) Antigene und Kopplung:
   Herkunft und Kopplung der synthetischen Peptide wie in Beispiel 6
b) Testdurchführung:
   Konventionell
   Wie in Beispiel 6 beschrieben, wurden 25 *µ*l der sensitivierten synthetischen Partikel und danach 5 *µ*l Serum/Plasma in die Inkubationskammer vorgefertigter Mikroröhrchen pipettiert. Nach 10 min Inkubation bei Raumtemperatur wurde für 10 min bei 85 g in einer für die Mikroröhrchen geeigneten Zentrifuge (ID-Centrifuge 12 S II, DiaMed, Cressier sur Morat) zentrifugiert.
   Modifiziert
   Wie in Beispiel 6 beschrieben, wurden 25 *µ*l der synthetischen Partikel und danach 5 *µ*l Serum/Plasma in die Inkubationskammer vorgefertigter Mikroröhrchen pipettiert. Nach 5 min Inkubation bei Raumtemperatur wurden die Karten in eine modifizierte ID-Zentrifuge (DiaMed, Cressier sur Morat) gegeben, mit der folgendes Spezial-Programm gefahren wurde:
   1) 5 s Zentrifugation, 30 g
   2) 5 min Pause, 0 g
   3) 10 min Zentrifugation, 85 g
c) Auswertung:
   Die Auswertung erfolgte wie in Beispiel 6. In Tests, welche mit Anti-IgG haltigen Gel-Matrizes operieren, ist eine allgemeine Verstärkung vor allem schwach positiver Ergebnisse zu beobachten.

## Patentansprüche

1. Verfahren zum Nachweis eines Analyten in einer Probeflüssigkeit durch Agglutination, wobei man die Probeflüssigkeit mit einem Agglutinationsreagenz und einer inerten Matrix in Kontakt bringt, die Reaktionsmischung dem Einwirken von Gravitation aussetzt und die Reaktion zwischen dem Analyten und dem Agglutinationsreagenz bestimmt,
**dadurch gekennzeichnet,**
daß man als Agglutinationsreagenz synthetische Partikel verwendet, die sich gegenüber der Matrix im wesentlichen wie Erythrozyten verhalten und einen mittleren Durchmesser ≤ 5 *µ* m und eine spezifische Dichte ≥ 1,1 g/cm³ aufweisen.

2. Verfahren nach Anspruch 1,
**dadurch gekennzeichnet,**
daß die synthetischen Partikel einen mittleren Durchmesser im Bereich von 1 - 5 *µ*m aufweisen.

3. Verfahren nach Anspruch 2,
**dadurch gekennzeichnet,**
daß die synthetischen Partikel einen mittleren Durchmesser im Bereich von 2 - 4 *µ*m aufweisen.

4. Verfahren nach Anspruch 1,
**dadurch gekennzeichnet,**
daß die synthetischen Partikel eine spezifische Dichte im Bereich von 1,1- 1,8 g/cm³ aufweisen.

5. Verfahren nach Anspruch 4,
**dadurch gekennzeichnet,**
daß die synthetischen Partikel eine spezifische Dichte im Bereich von 1,15 - 1,4 g/cm³ aufweisen.

6. Verfahren nach einem der Ansprüche 1-5,
**dadurch gekennzeichnet,**
daß man synthetische Partikel aus organischen Polymeren oder Copolymeren verwendet.

7. Verfahren nach Anspruch 6,
**dadurch gekennzeichnet,**
daß man synthetische Partikel aus Styrol oder Styrolderivaten verwendet.

8. Verfahren nach einem der Ansprüche 1-7,
**dadurch gekennzeichnet,**
daß man gefärbte Partikel verwendet.

9. Verfahren nach Anspruch 8,
**dadurch gekennzeichnet,**
daß man rot gefärbte Partikel verwendet.

10. Verfahren nach einem der Ansprüche 1-9,
**dadurch gekennzeichnet,**
daß auf der Oberfläche der Partikel Ligandenmoleküle immobilisiert sind, die mit dem zu bestimmenden Analyten bindefähig sind.

11. Verfahren nach Anspruch 10,
**dadurch gekennzeichnet,**
daß die Ligandenmoleküle über adsorptive, kovalente oder hochaffine Wechselwirkungen immobilisiert sind.

12. Verfahren nach Anspruch 10 oder 11,
**dadurch gekennzeichnet,**
daß auf der Oberfläche der Partikel Peptide, Proteine, Nukleinsäuren, Nukleinsäureanaloga, Saccharide, Lipide, Hormone oder Metaboliten über kovalente oder hochaffine Wechselwirkungen immobilisiert sind.

13. Verfahren nach Anspruch 10 oder 11,
**dadurch gekennzeichnet,**
daß auf der Oberfläche der Partikel Zellmembranen, Fragmente von Zellmembranen oder Lysate von Zellen oder Pathogenen durch adsorptive Wechselwirkungen gebunden sind.

14. Verfahren nach einem der Ansprüche 1-13,
**dadurch gekennzeichnet,**
daß die Bestimmung des Analyten durch eine Immunreaktion erfolgt.

15. Verfahren nach Anspruch 14,
**dadurch gekennzeichnet,**
daß der Analyt ein Antikörper ist.

16. Verfahren nach Anspruch 15,
**dadurch gekennzeichnet,**
daß ein klassenspezifischer Nachweis von Antikörpern durchgeführt wird.

17. Verfahren nach Anspruch 14,
**dadurch gekennzeichnet,**
daß der Analyt ein Antigen ist.

18. Verfahren nach einem der Ansprüche 1-13,
**dadurch gekennzeichnet,**
daß die Bestimmung des Analyten durch eine Nucleinsäure-Hybridisierungsreaktion erfolgt.

19. Verfahren nach einem der Ansprüche 1-18,
**dadurch gekennzeichnet,**
daß man eine partikuläre Matrix verwendet.

20. Verfahren nach Anspruch 19,
**dadurch gekennzeichnet,**
daß der mittlere Durchmesser der Matrixpartikel im Bereich von 10-200 *µ* m ist.

21. Verfahren nach einem der Ansprüche 1-20,
**dadurch gekennzeichnet,**
daß mehrere Analyten gleichzeitig bestimmt werden, wobei für jeden Analyten ein unterschiedlich gefärbtes Agglutinationsreagenz eingesetzt wird.

22. Verfahren nach einem der Ansprüche 1-21,
**dadurch gekennzeichnet,**
daß die Reaktionsmischung Detergenzien oder/und Mucine enthält.

23. Verfahren nach einem der Ansprüche 1-22,
**dadurch gekennzeichnet,**
daß die Reaktionsmischung ein Reduktionsmittel enthält.

24. Verfahren nach einem der Ansprüche 1-23,
**dadurch gekennzeichnet,**
daß das Einwirken von Gravitation auf die Reaktionsmischung eine Zentrifugation umfasst.

25. Verfahren nach Anspruch 24,
**dadurch gekennzeichnet,**
daß die Zentrifugation in mehreren Intervallen durchgeführt wird.

26. Reagenzienkit zum Nachweis eines Analyten in einer Probeflüssigkeit, umfassend
(a) mindestens ein Reaktionsgefäß, das eine inerte Matrix enthält und
(b) mindestens eine Art von synthetischen Partikeln mit einem mittleren Durchmesser ≤ 5 *µ*m und einer spezifischen Dichte ≥ 1,1 g/cm³, auf deren Oberfläche Ligandenmoleküle immobilisiert sind.

27. Reagenzienkit nach Anspruch 26,
**dadurch gekennzeichnet,**
daß mehrere Reaktionsgefäße auf einer Karte oder Scheibe angeordnet sind.

## Claims

1. Process for detecting an analyte in a sample fluid by agglutination, in which the sample fluid is brought into contact with an agglutination reagent and an inert matrix, the reaction mixture is exposed to the action of gravitation and the reaction between the analyte and the agglutination reagent is determined, characterised in that synthetic particles are used as agglutination reagent, which behave substantially as erythrocytes relative to the matrix and have a mean diameter ≤ 5 µm and a specific density ≥ 1.1 g/cm³.

2. Process according to claim 1, characterised in that the synthetic particles have a mean diameter in the range of 1 to 5 µm.

3. Process according to claim 2, characterised in that the synthetic particles have a mean diameter in the range of 2 to 4 *µ*m.

4. Process according to claim 1, characterised in that the synthetic particles have a specific density in the range of 1.1 to 1.8 g/cm³.

5. Process according to claim 4, characterised in that the synthetic particles have a specific density in the range of 1.15 to 1.4 g/cm³.

6. Process according to one of claims 1 to 5, characterised in that synthetic particles composed of organic polymers or copolymers are used.

7. Process according to claim 6, characterised in that synthetic particles composed of styrene or styrene derivatives are used.

8. Process according to one of claims 1 to 7, characterised in that coloured particles are used.

9. Process according to claim 8, characterised in that red-coloured particles are used.

10. Process according to one of claims 1 to 9, characterised in that ligand molecules capable of bonding to the analyte to be determined are immobilised on the surface of the particles.

11. Process according to claim 10, characterised in that the ligand molecules are immobilised via adsorptive, covalent or high-affinity interactions.

12. Process according to claim 10 or 11, characterised in that peptides, proteins, nucleic acids, nucleic acid analogues, saccharides, lipids, hormones or metabolites are immobilised via covalent or high-affinity interactions on the surface of the particles.

13. Process according to claim 10 or 11, characterised in that cell membranes, fragments of cell membranes or lysed cell materials or pathogens are bound by adsorptive interactions on the surface of the particles.

14. Process according to one of claims 1 to 13, characterised in that the analyte is determined by an immune reaction.

15. Process according to claim 14, characterised in that the analyte is an antibody.

16. Process according to claim 15, characterised in that class-specific detection of antibodies is carried out.

17. Process according to claim 14, characterised in that the analyte is an antigen.

18. Process according to one of claims 1 to 13, characterised in that the analyte is determined by a nucleic acid hybridisation reaction.

19. Process according to one of claims 1 to 18, characterised in that a particulate matrix is used.

20. Process according to claim 19, characterised in that the mean diameter of the matrix particles is in the range of 10 to 200 µm.

21. Process according to one of claims 1 to 20, characterised in that a plurality of analytes are determined simultaneously, a differently coloured agglutination reagent being used for each analyte.

22. Process according to one of claims 1 to 21, characterised in that the reaction mixture contains detergents or/and mucins.

23. Process according to one of claims 1 to 22, characterised in that the reaction mixture contains a reducing agent.

24. Process according to one of claims 1 to 23, characterised in that the effect of gravitation on the reaction mixture comprises centrifugation.

25. Process according to claim 24, characterised in that centrifugation is carried out at a plurality of intervals.

26. Reagents kit for detecting an analyte in a sample fluid, comprising
(a) at least one reaction vessel containing an inert matrix and
(b) at least one type of synthetic particles with a mean diameter ≤ 5 µm and a specific density ≥ 1.1 g/cm³ having ligand molecules immobilised on its surface.

27. Reagents kit according to claim 26, characterised in that a plurality of reaction vessels are arranged on a card or disc.

## Revendications

1. Procédé de détection d'un analyte dans un échantillon liquide par agglutination, dans lequel on met l'échantillon liquide en contact avec un réactif d'agglutination et une matrice inerte, on soumet le mélange réactionnel à l'action d'une gravitation et on détermine la réaction entre l'analyte et le réactif d'agglutination, caractérisé en ce que l'on utilise comme réactif d'agglutination des particules synthétiques qui se comportent vis-à-vis de la matrice essentiellement comme des érythrocytes et qui ont un diamètre moyen inférieur ou égal à 5 µm et une densité spécifique supérieure ou égale à 1,1 g/cm³.

2. Procédé selon la revendication 1, caractérisé en ce que les particules synthétiques ont un diamètre moyen compris entre 1 et 5 µm.

3. Procédé selon la revendication 2, caractérisé en ce que les particules synthétiques ont un diamètre moyen compris entre 2 et 4 µm.

4. Procédé selon la revendication 1, caractérisé en ce que les particules synthétiques ont une densité spécifique comprise entre 1,1 et 1,8 g/cm³.

5. Procédé selon la revendication 4, caractérisé en ce que les particules synthétiques ont une densité spécifique comprise entre 1,15 et 1,4 g/cm³.

6. Procédé selon l'une des revendications 1 à 5, caractérisé en ce que l'on utilise des particules synthétiques de polymères ou de copolymères organiques.

7. Procédé selon la revendication 6, caractérisé en ce que l'on utilise des particules synthétiques de styrène ou de dérivés de styrène.

8. Procédé selon l'une des revendications 1 à 7, caractérisé en ce que l'on utilise des particules colorées.

9. Procédé selon la revendication 8, caractérisé en ce que l'on utilise des particules colorées en rouge.

10. Procédé selon l'une des revendications 1 à 9, caractérisé en ce que des molécules de ligand capables de se lier avec l'analyte à déterminer sont immobilisées sur la surface des particules.

11. Procédé selon la revendication 10, caractérisé en ce que les molécules de ligand sont immobilisées par l'intermédiaire d'interactions d'adsorption, de covalence ou de haute affinité.

12. Procédé selon la revendication 10 ou 11, caractérisé en ce que des peptides, des protéines, des acides nucléiques, des analogues d'acides nucléiques, des saccharides, des lipides, des hormones ou des métabolites sont immobilisés sur la surface des particules par l'intermédiaire d'interactions de covalence ou de haute affinité.

13. Procédé selon la revendication 10 ou 11, caractérisé en ce que des membranes cellulaires, des fragments de membranes cellulaires ou des lysats de cellules ou de pathogènes sont liés à la surface des particules par des interactions d'adsorption.

14. Procédé selon l'une des revendications 1 à 13, caractérisé en ce que la détermination de l'analyte s'effectue par une réaction immunologique.

15. Procédé selon la revendication 14, caractérisé en ce que l'analyte est un anticorps.

16. Procédé selon la revendication 15, caractérisé en ce que l'on effectue une détection spécifique de la classe d'anticorps.

17. Procédé selon la revendication 14, caractérisé en ce que l'analyte est un antigène.

18. Procédé selon l'une des revendications 1 à 13, caractérisé en ce que la détermination de l'analyte s'effectue par une réaction d'hybridation d'acides nucléiques.

19. Procédé selon l'une des revendications 1 à 18, caractérisé en ce que l'on utilise une matrice sous forme de particules.

20. Procédé selon la revendication 19, caractérisé en ce que le diamètre moyen des particules de la matrice est compris entre 10 et 200 µm.

21. Procédé selon l'une des revendications 1 à 20, caractérisé en ce que l'on détermine plusieurs analytes en même temps en utilisant pour chaque analyte un réactif d'agglutination de couleur différente.

22. Procédé selon l'une des revendications 1 à 21, caractérisé en ce que le mélange réactionnel contient des détergents et/ou des mucines.

23. Procédé selon l'une des revendications 1 à 22, caractérisé en ce que le mélange réactionnel contient un agent réducteur.

24. Procédé selon l'une des revendications 1 à 23, caractérisé en ce que l'action de la gravitation sur le mélange réactionnel comprend une centrifugation.

25. Procédé selon la revendication 24, caractérisé en ce que l'on effectue la centrifugation en plusieurs intervalles.

26. Trousse de réactifs pour la détection d'un analyte dans un échantillon liquide, comprenant
(a) au moins un récipient de réaction contenant une matrice inerte et
(b) au moins un type de particules synthétiques ayant un diamètre moyen inférieur ou égal à 5 µm et une densité spécifique supérieure ou égale à 1,1 g/cm³, sur la surface desquelles sont immobilisées des molécules de ligand.

27. Trousse de réactifs selon la revendication 26, caractérisée en ce que plusieurs récipients de réaction sont disposés sur une carte ou un disque.
